# FASCICULE DE BREVET EUROPEEN

(11) **EP 1 539 804 B1**
(45) Date de publication et mention de la délivrance du brevet: **03.01.2007**
(21) Numéro de dépôt: 03797355.9
(22) Date de dépôt: 19.09.2003
(51) Int. Cl.: C07K 9/00, A61K 38/04, G01N 33/68, C07K 7/64

(54) **SYNTHESE ET CARACTERISATION DE NOUVEAUX SYSTEMES DE GUIDAGE ET DE VECTORISATION DE MOLECULES D'INTERET THERAPEUTIQUE VERS DES CELLULES CIBLES.**
SYNTHESE UND CHARAKTERISIERUNG NEUER SYSTEME ZUM HINFÜHREN UND ZUR VEKTORISIERUNG VON MOLEKÜLEN VON THERAPEUTISCHEM INTERESSE ZU TARGETZELLEN
SYNTHESIS AND CHARACTERIZATION OF NOVEL SYSTEMS FOR GUIDANCE AND VECTORIZATION OF MOLECULES OF THERAPEUTIC INTEREST TOWARDS TARGET CELLS

(30) Priorité: 19.09.2002 FR 0211614; 19.09.2002 US 411845 P
(43) Date de publication de la demande: 15.06.2005
(73) Titulaire: CENTRE NATIONAL DE LA RECHERCHE SCIENTIFIQUE (CNRS), 75794 Paris Cedex 16 (FR); UNIVERSITE JOSEPH FOURIER (GRENOBLE 1), F-38041 Grenoble Cédex 09 (FR); INSERM, 75654 Paris Cédex 13 (FR)
(72) Inventeur: DUMY, Pascal, F- 38580 Allevard (FR); FAVROT, Marie-Christine, F- 38700 Corenc (FR); BOTURYN, Didier, F-38000 Grenoble (FR); COLL, Jean-Luc, F- Claix (FR)
(74) Mandataire: Breese, Pierre
(86) Numéro de dépôt international: PCT/FR2003/002773
(87) Numéro de publication internationale: WO 2004/026894

(56) Documents cités:
- P DUMY ET AL.: "A convenient synthesis of cyclic peptides as regioselectively addressable functionalized templates (RAFT)" TETRAHEDRON LETTERS., vol. 38, no. 8, 1995, pages 1255-1258, XP002251829 ELSEVIER SCIENCE PUBLISHERS, AMSTERDAM., NL ISSN: 0040-4039
- F PERI ET AL.: "Chemo- and stereoselective glycosylation of hydroxylamino derivatives: a versatile approach to glycoconjugates" TETRAHEDRON., vol. 54, 1998, pages 12269-12278, XP002251830 ELSEVIER SCIENCE PUBLISHERS, AMSTERDAM., NL ISSN: 0040-4020
- O RENAUDET & P DUMY: "Expedient synthesis of aminooxylated-carbohydrates for chemoselective access of glycoconjugates" TETRAHEDRON LETTERS., vol. 42, 2001, pages 7575-7578, XP002251831 ELSEVIER SCIENCE PUBLISHERS, AMSTERDAM., NL ISSN: 0040-4039
- O RENAUDET & P DUMY: "Chemoselectively template-assembled glycoconjugates as mimics for multivalent presentation of carbohydrates" ORGANIC LETTERS., vol. 5, no. 3, 2003, pages 243-246, XP002251832 ACS, WASHINGTON, DC., US ISSN: 1523-7060
- D FORGET ET AL. : "3-oligonucleotides conjugation via chemoselective oxime bond formation" TETRAHEDRON LETTERS., vol. 42, no. 52, 2001, pages 9171-9174, XP004328105 ELSEVIER SCIENCE PUBLISHERS, AMSTERDAM., NL ISSN: 0040-4039

## Description

La présente invention concerne la synthèse et la caractérisation de nouveaux systèmes de guidage et de vectorisation de molécules d'intérêt thérapeutique vers des cellules cibles.

Plus précisément, l'invention s'intéresse à des complexes moléculaires capables de combiner plusieurs molécules fonctionnelles, possédant des propriétés prédéfinies de reconnaissances ou effectrices, à leur procédé de préparation et à leur utilisation thérapeutique ou comme outils de diagnostic.

La littérature décrit de nombreux procédés d'obtention de complexes bio-conjugués polyfonctionnels monovalents (Lemieux et al. *Trends in Biotechnology,* 1998, 16, 506-513). Toutefois, ces complexes suscitent une réponse biologique relativement faible. Des systèmes multivalents ont alors été préparés (Tam et al., *Biomedical Peptides, Proteins & Nucleic Acids,* 1995, 1, 123-132). Des études ont démontré que les complexes polyvalents étaient, de manière générale, de bien meilleurs outils biologiques que les complexes bio-conjugués monovalents (Grubbs, R.H. et al., *J.Am. Chem. Soc.,* 2001,123,1275-1279).

L'intérêt de tels complexes bio-conjugués est d'associer les propriétés propres d'au moins deux classes de molécules différentes. Toutefois, une des difficultés qui se présente est l'interaction potentielle que peuvent présenter les différentes molécules d'un bio-conjugué. Cette interaction peut modifier les propriétés résultant de la bio-conjugaison. Une solution à ce problème consiste à séparer spatialement les points de bio-conjugaison, ce qui a conduit au développement de gabarit ou de châssis moléculaires adressables. L. Scheibler, P. Dumy et al., *Tetrahedron,* 1998, 54, 3725-3734, décrit la synthèse et la caractérisation de châssis moléculaires fonctionnalisés avec des groupes de coordination et des chaînes alcanes.

Une autre difficulté est la fixation sur le châssis des molécules d'intérêt. L. Scheibler, P. Dumy et al., *Angew. Chem. Int. Ed. ,* 1999, 38, 696-699, décrit un châssis fonctionnalisé chimiosélectivement sur une face au moyen d'une liaison oxime.

Les enseignements de l'état de la technique ne permettent toutefois pas de synthétiser un châssis fonctionnalisé sur les deux faces, une au moins des fonctionnalisations étant chimiosélective, chaque face portant des molécules d'intérêt thérapeutique ou de diagnostic ou de marquage.

L'invention a donc pour objet un procédé de préparation d'un cyclopeptide homodétique greffé sur ses deux faces.

Plus précisément, l'invention a pour objet un procédé de préparation d'un cyclopeptide homodétique greffé formant un châssis définissant deux faces, une face dite supérieure et une face dite inférieure, lesdites deux faces étant toutes les deux greffées, caractérisé en ce que l'on synthétise un peptide linéaire, ladite synthèse étant effectuée à partir d'acides aminés modifiés ou non, certains d'entre eux portant des groupes protecteurs orthogonaux, on effectue une cyclisation intramoléculaire du peptide linéaire obtenu, on substitue tout ou partie des groupes protecteurs orthogonaux par un précurseur protégé, on greffe sur l'une et/ou l'autre face dudit châssis, par une liaison oxime, au moins une molécule d'intérêt.

La présente invention concerne donc un procédé de préparation du châssis moléculaire d'une molécule polyfonctionnelle, obtenue en quatre étapes : (1) la synthèse d'un peptide linéaire ; (2) la cyclisation intramoléculaire de ce peptide linéaire de manière à obtenir un châssis moléculaire ; (3) la fonctionnalisation de ce châssis ; (4) le greffage d'au moins une molécule d'intérêt sur l'une et/ou l'autre face de ce châssis.

Les acides aminés utilisés pour la synthèse peptidique sont de toute nature, y compris les acides aminés de la série (D), les acides aminés de la série (L), ainsi que tout acide aminé modifié, les acides aminés étant naturels ou synthétiques. Certains de ces acides aminés sont substitués par des groupes protecteurs orthogonaux. Les groupes protecteurs orthogonaux sont des groupes chimiques orientés perpendiculairement par rapport au plan médian du châssis ; ils masquent la réactivité d'un atome ou d'un groupe d'atome et leur élimination chimique n'affecte pas les autres groupes protecteurs de nature différente présents au sein de la molécule. Leur choix dépend de la nature de l'acide aminé et du châssis désiré.

Suivant un premier mode de réalisation de l'invention, la synthèse du peptide linéaire, effectuée sur phase solide, est initiée à partir d'un résidu de glycine dont la fonction carboxylique est ancrée à une résine, et la cyclisation du peptide linéaire obtenu est effectuée en solution après libération de la résine. La stratégie d'élongation d'un tel peptide linéaire est décrite dans l'état de la technique (P. Dumy et al., Tetrahedron Lett. 1995, 36, 1255-1258). L'initiation de la synthèse par un résidu de glycine permet d'éviter tout risque d'épimérisation durant l'étape suivante de cyclisation. De préférence, la glycine est reliée à la résine par sa fonction carboxylique. Sa fonction alpha amine est protégée par un groupe protecteur permettant par synthèse l'élongation du peptide. À l'issue de la synthèse du peptide linéaire, la fonction alpha aminoterminale du peptide est libérée du groupe protecteur au cours d'une première étape et la fonction carboxyterminale est libérée de la résine au cours d'une seconde étape de libération du peptide linéaire protégé, les chaînes latérales restant protégées par leur groupe protecteur orthogonal. Les fonctions chimiques N- et C-terminales libérées du peptide linéaire solubilisé réagissent pour former une liaison peptidique intramoléculaire au cours d'une étape de cyclisation intramoléculaire effectuée en solution.

Suivant un second mode de réalisation, la synthèse du peptide linéaire puis sa cyclisation sont entièrement effectuées sur phase solide. Dans ce mode de réalisation, la synthèse du peptide linéaire est initiée par un résidu d'acide aminé dont la chaîne latérale est ancrée à une résine, laissant sa fonction carboxylique libre d'ancrage. La fonction alpha amine et la fonction carboxylique de cet acide aminé sont protégées chacune par un groupe protecteur, les deux groupes protecteurs étant orthogonaux entre eux.

À l'issue de la synthèse du peptide linéaire, les fonctions alpha aminoterminale et carboxyterminale sont libérées sélectivement de leur groupe protecteur respectif et le peptide linéaire obtenu reste lié à la résine par la chaîne latérale du premier résidu d'acide aminé. Les fonctions chimiques terminales du peptide linéaire obtenu sont alors libres pour pouvoir réagir entre elles et former un cycle intramoléculaire au cours d'une étape de cyclisation intramoléculaire effectuée sur phase solide.

Dans ce mode de réalisation, le procédé est avantageusement, entièrement ou partiellement automatisé sur robot synthétiseur de peptides.

Avantageusement, le cyclopeptide est formé de 5, 10 ou 14 résidus d'acides aminés, de préférence 10 acides aminés formant un cyclodécapeptide. Le cyclopeptide cyclisé selon l'invention présente au moins un coude, préférentiellement deux coudes. Certains cyclopeptides selon l'invention présentent une symétrie centrale.

Suivant un mode de réalisation préféré, le cyclopeptide présente 10 ou 14 résidus d'acides aminés et forme deux coudes, chaque coude étant formé par une combinaison (L)Pro-(D)AA ou (D)Pro-(L)AA, AA étant un acide aminé et de préférence la glycine, les deux coudes étant séparés par trois et/ou cinq résidus d'acides aminés.

La présence du réside proline au niveau du coude est justifiée par le fait que la proline montre une configuration spatiale caractéristique, en comparaison des autres acides aminés, du fait de sa structure cyclique. Cette caractéristique confère une restriction conformationnelle au squelette peptidique en comparaison à celle adoptée avec des acides aminés autre que la proline ou ses dérivés. Cette restriction est, notamment, à l'origine des coudes dans les structures polypeptidiques secondaires et supersecondaires.

L'autre résidu d'acide aminé du coude, ci-dessus représenté par le sigle AA, est préférentiellement un résidu d'acide aminé autre que celui de la proline et de stéréochimie opposée et très préférentiellement le résidu glycine.

Les coudes sont séparés par des résidus d'acides aminés, préférentiellement un nombre impair de résidu d'acides aminés et très préférentiellement trois et/ou cinq acides aminés pour un cyclodécapeptide et un cyclotérapeptide respectivement.

Les cyclopeptides à deux coudes et ayant un nombre pair de résidus d'acides aminés présentent un plan médian qui définit une face dite supérieure et une face dite inférieure.

De préférence, les trois et/ou cinq résidus d'acides aminés ont chacun une fonction chimique protégée orthogonalement par un groupe protecteur. Les groupes protecteurs des chaînes latérales de ces acides aminés se dirigent alternativement de part et d'autre du plan médian dudit châssis et définissent une face dite inférieure et supérieure par rapport à ce plan.

Ces résidus d'acides aminés sont préférentiellement des résidus d'acides aminés portant des fonctions chimiques du type -NH2, -SH ou -COOH. Suivant un mode de réalisation particulier de l'invention, ces trois ou cinq résidus d'acides aminés sont préférentiellement des acides aminés à chaîne latérale amine, et très préférentiellement, la lysine.

Suivant un mode de réalisation particulier de l'invention, les groupes protecteurs orthogonaux des résidus d'acides aminés centraux sont identiques entre eux, les groupes protecteurs orthogonaux des résidus d'acides aminés autres que centraux sont identiques entre eux, les groupes protecteurs orthogonaux des résidus d'acides aminés centraux, d'une part, et les groupes protecteurs orthogonaux des autres résidus d'acides aminés, d'autre part, sont différents les uns des autres.

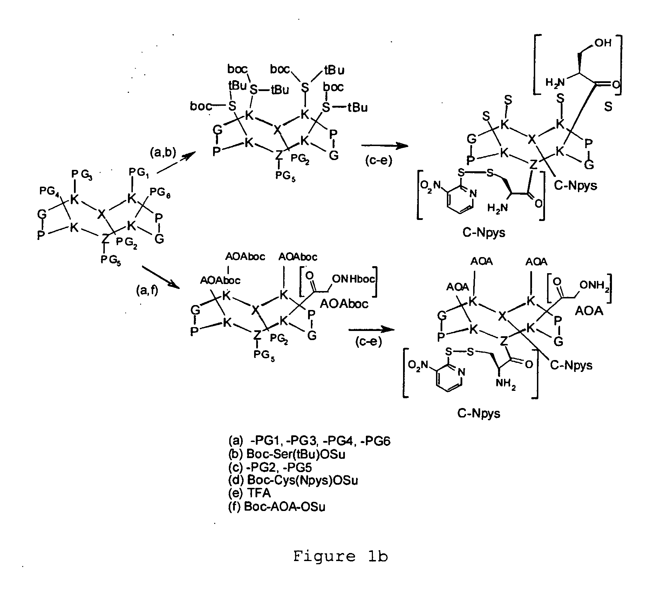

Suivant un mode de réalisation particulier de l'invention, on débute le greffage du châssis en substituant des groupes protecteurs orthogonaux du châssis par un précurseur protégé de la fonction oxyamine ou un précurseur masqué de la fonction aldéhyde, en particulier du type α-oxoaldéhyde, ou par un précurseur protégé de la fonction thiol ou par un marqueur. Suivant une première variante de l'invention, le précurseur protégé de la fonction thiol est un dérivé disulfure dissymétrique de la cystéine et en particulier un groupe Npys (figure 1b)

Suivant une autre variante de l'invention, ce précurseur protégé est l'acide 2-oxyamino-acétique protégé (AOA) sur l'azote ou encore un dérivé de la sérine, précurseur de la fonction α-oxoaldéhyde, dont les fonctions amine et hydroxyle sont protégées, et dont la déprotection puis le clivage oxydatif libère le groupement aldéhyde, et de préférence est Boc-Ser(tBu)OH.

Suivant un premier mode de réalisation, l'on effectue en premier lieu la substitution des groupes protecteurs orthogonaux de la face inférieure par un marqueur, de préférence la biotine ou la fluorescéine, puis dans une deuxième étape, on substitue les groupes protecteurs orthogonaux de la face supérieure du châssis par un précurseur protégé de la fonction oxyamine ou de la fonction α-oxoaldéhyde. On fait réagir ensuite la fonction oxyamine ou α-oxoaldéhyde du précurseur, préalablement déprotégé, avec une molécule d'intérêt ou une molécule intermédiaire portant une fonction respectivement α-oxoaldéhyde ou oxyamine. Dans ce mode de réalisation, la substitution de la face inférieure est classique, tandis que la substitution de la face supérieure est chimiosélective.

Suivant un second mode de réalisation, l'on effectue en premier lieu la substitution des groupes protecteurs orthogonaux de la face inférieure du châssis par un précurseur protégé de la fonction oxyamine puis dans une deuxième étape on substitue les groupes protecteurs orthogonaux de la face supérieure du cyclopeptide par un précurseur protégé de la fonction α-oxoaldéhyde.

Suivant un troisième mode de réalisation de l'invention l'on effectue en premier lieu la substitution des groupes protecteurs orthogonaux de la face supérieure du châssis par un précurseur protégé de la fonction oxyamine puis dans une deuxième étape on substitue les groupes protecteurs orthogonaux de la face inférieure du cyclopeptide par un précurseur protégé de la fonction α-oxoaldéhyde.

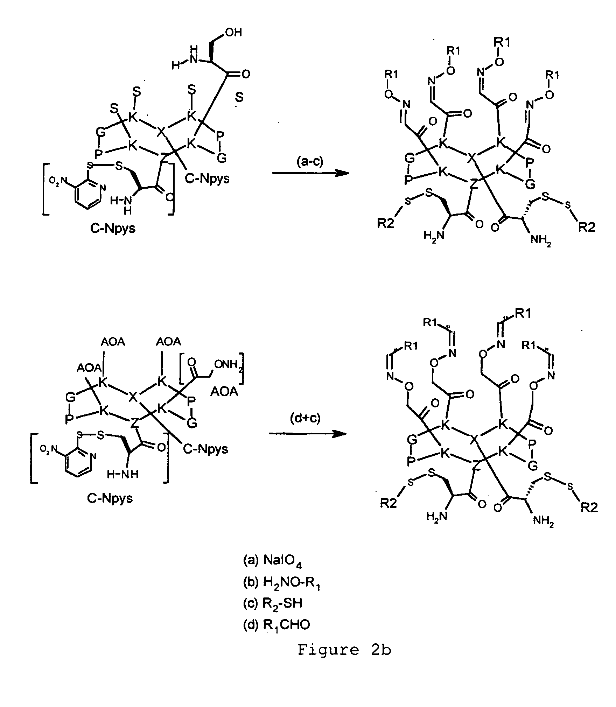

Suivant un quatrième mode de réalisation de l'invention, on effectue la substitution des groupes protecteurs orthogonaux d'une face du châssis par un précurseur protégé de la fonction aldéhyde ou oxyamine puis la substitution des groupes protecteurs orthogonaux de l'autre face du châssis par un précurseur protégé de la fonction thiol. On effectue ensuite le greffage des molécules d'intérêt en faisant réagir des groupes fonctionnels complémentaires. Dans ce mode de réalisation, on effectue en premier lieu le greffage des molécules d'intérêt portant un précurseur aldéhyde ou oxyamine sur la face du châssis portant un précurseur oxyamine ou aldéhyde respectivement puis on fait réagir l'autre face du châssis sous sa forme thiol libre ou sous forme de disulfure dissymétrique activé avec une seconde molécule d'intérêt portant une fonction disulfure dissymétrique activé ou thiol libre respectivement. Cette seconde molécule est alors attachée sur le châssis via une agrafe disulfure. Les molécules d'intérêt peuvent être un peptide, une protéine, un oligosaccharide, un acide nucléique, une molécule organique, une molécule inorganique (Figures 1b et 2b). Avantageusement cette agrafe peut permettre le relargage intra-cellulaire de la molécule d'intérêt afin qu'elle puisse jouer son rôle biologique.

Dans les modes de réalisation ci-dessus, l'on fait réagir les fonctions actives, notamment oxyamine ou α-oxoaldéhyde des précurseurs, préalablement déprotégées, avec une ou plusieurs molécules d'intérêt ou une molécule intermédiaire portant une fonction complémentaire, dans notre exemple respectivement α-oxoaldéhyde ou oxyamine. De préférence, l'on fait réagir la fonction oxyamine du précurseur située sur le châssis avec une molécule d'intérêt portant une fonction α-oxoaldéhyde, puis on oxyde le précurseur de la fonction α-oxoaldéhyde situé sur le châssis et l'on poursuit la réaction avec une mise en contact du châssis avec au moins une molécule d'intérêt ou une molécule intermédiaire portant une fonction oxyamine.

La ou les molécules intermédiaires portent d'une part une fonction oxyamine capable de réagir avec la ou les fonctions α-oxoaldéhydes situées sur le châssis, et portent d'autre part un précurseur d'au moins une fonction α-oxoaldéhyde.

La ou les molécules d'intérêt sont identiques ou différentes les unes des autres.

Avantageusement, la molécule d'intérêt est un acide nucléique, un peptide, un oligosaccharide, ou une molécule organique. Toute molécule d'intérêt thérapeutique ou de diagnostic portant une fonction oxyamine ou α-oxoaldéhyde peut être greffée sur le châssis.
Suivant une première variante de l'invention, on greffe sur une face du châssis avec des peptides dérivés du cyclo (RGDfK) et du cyclo (RGDyK) ligands de l'intégrine αVβ3, afin de diriger la molécule vers les tissus exprimant ce récepteur et de modifier des effets biologiques dans un but thérapeutique ou de diagnostic.
◆ Si le but est thérapeutique, on greffe l'autre face du châssis selon l'invention avec un peptide apotogène du type (KLAKKLAK), ou une molécule organique connu du type (doxorobucine) ou une protéine toxique intracellulairement (ricin-A, galénine...)
◆ Si le but est de diagnostic in vitro et in vivo on greffe l'autre face du châssis avec un ou des chromophore(s), une ou des biotine(s) ; un ou des fluorophore(s), une ou des radio-émetteur(s) ou un groupe précurseur (chimique ou ligand)

Suivant une seconde variante de l'invention, on greffe une face du châssis avec des dérivés carbohydrates pour le ciblage de leur récepteur transmembranaire de type lectine notamment récepteur à mannose, récepteur à galactose, récepteur asialo-protéine, récepteur transporteur du glucose GLUT... dans un but thérapeutique ou de diagnostic.
◆ Si le but est thérapeutique, on greffe l'autre face du châssis selon l'invention avec un ou des peptides(s) épitope(s) T-dépendant, avec un ou des peptides apotogènes du type (KLAKKLAK), ou une molécule organique connu du type (doxorobucine) ou une protéine toxique intracellulairement (ricin-A, galénine...)
◆ Si le but est de diagnostic in vitro et in vivo on greffe l'autre face du châssis avec un ou des chromophore(s), une ou des biotine(s) ; un ou des fluorophore(s), une ou des radio-émetteur(s) ou un groupe précurseur (chimique ou ligand)

Suivant une troisième variante de l'invention, on greffe une face du châssis avec des épitopes B-dépendant de type peptide ou carbohydrate plus particulièrement marqueur de tumeur (Tn, sTn, Tf), un ou des épitopes T-dépendants (peptides Th1 ou Th2) et un immunoadjuvant afin de susciter la réponse cellulaire dans une but de vaccination.

Suivant une quatrième variante de l'invention, on fonctionnalise les surfaces dans le but de leur conférer des propriétés de reconnaissance utile dans les systèmes miniaturisés de type bio-puce.

Avantageusement, le procédé selon l'invention, lorsque la synthèse et la cyclisation sont réalisées en phases solides, est entièrement ou partiellement automatisé sur robot synthétiseur de peptide.

L'invention a également pour objet un cyclopeptide homodétique greffé caractérisé en ce qu'il est obtenu par le procédé selon l'invention.

Un autre objet de l'invention est une composition thérapeutique ou de diagnostic caractérisée en ce qu'elle comprend un cyclopeptide homodétique greffé obtenu par le procédé selon l'invention.

L'invention concerne également l'utilisation d'un cyclopeptide homodétique greffé obtenu par le procédé selon l'invention ou d'une composition le contenant, pour la fabrication d'un médicament destiné à soigner le cancer. L'invention concerne également l'utilisation d'un cyclopeptide homodétique greffé obtenu par le procédé selon l'invention ou d'une composition le contenant, comme outil de diagnostic du cancer.

L'invention concerne également l'utilisation d'un cyclopeptide homodétique greffé obtenu par le procédé selon l'invention ou d'une composition le contenant, pour le diagnostic et/ou la suppression de la néoangiogénèse.

L'invention concerne également l'utilisation d'un cyclopeptide homodétique greffé obtenu par le procédé selon l'invention ou d'une composition le contenant, de châssis pour fonctionnaliser des surfaces dans le but de leur conférer des propriétés de reconnaissance utile dans les systèmes miniaturisés de type bio-puce.

L'utilisation de surface comme interface fonctionnelle douée de propriétés de reconnaissance représente une stratégie de choix pour la conception de ces systèmes qui est transférable avec une large gamme de technologie permettant leur miniaturisation micro- ou nanométrique ainsi que leur connectique.

D'une part, la fonctionnalisation de la surface hôte par un des partenaires impliqué dans la reconnaissance (sonde : molécules, biomolécules , cellules) est une étape clé pour réaliser cette interaction moléculaire à l'interface de la surface et de l'analyte. Cet adressage doit être suffisamment doux pour préserver l'intégrité et les propriétés moléculaires de la sonde, reproductible pour être exploitable et spatialement contrôlé pour permettre la détection de différentes cibles en parallèle. D'autre part, la fonctionnalisation peut conduire également à la transduction de la reconnaissance sonde-cible en un signal mesurable et quantifiable permettant la détection de la cible. L'intérêt majeur de cette stratégie réside dans l'absence de marquage de la cible qui permet la généralisation de l'approche à toutes catégories de cibles.

Dans ce cadre le procédé de l'invention permet d'utiliser une face du châssis pour l'adressage de la surface par exemple en utilisant des greffons thiols ou par électropolymérisation en utilisant des pendants de type pyrrole. L'autre face est utilisée selon le procédé de l'invention pour le greffage de molécule d'intérêt sur la surface. Avantageusement, l'utilisation de précurseur de la fonction α-oxoaldéhyde protégé par une groupe photolabile du type **NVOC** permet l'adressage spatialement contrôlé de la surface par une molécule d'intérêt. Le procédé de l'invention est largement plus avantageux pour la fonctionnalisation de surface que les méthodes classiquement utilisées.

D'autres modes de réalisation du châssis et de son greffage selon l'invention sont représentés dans la description détaillée ci-dessous, qui se lit en regard des figures et illustre non limitativement l'invention.

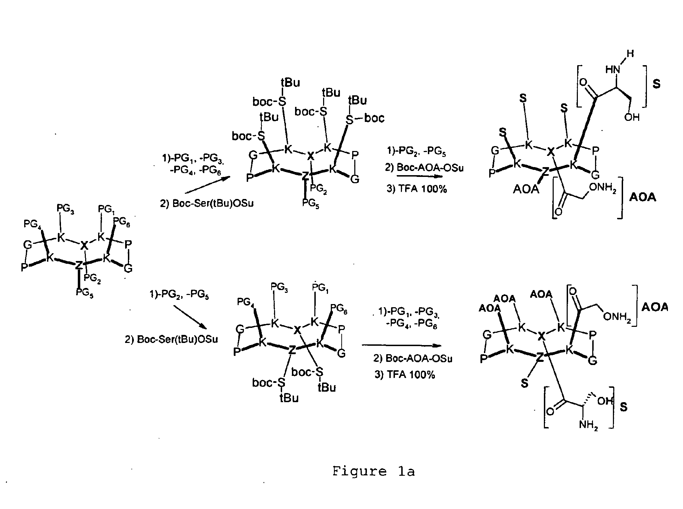

La figure 1a illustre le schéma de préparation de cyclodécapeptides selon l'invention comportant, d'une part sur la face supérieure la sérine précurseur de la fonction α-oxoaldéhyde et sur la face inférieure la fonction oxyamine, et d'autre part sur la face supérieure la fonction oxyamine et sur la face inférieure la sérine précurseur de la fonction α-oxoaldéhyde.

La figure 1b illustre le schéma de préparation de cyclodécapeptides selon l'invention comportant, sur la face supérieure, soit la sérine précurseur de la fonction α-oxoaldéhyde soit la fonction α-oxoaldéhyde, et d'autre part sur la face inférieure la fonction C-Npys.

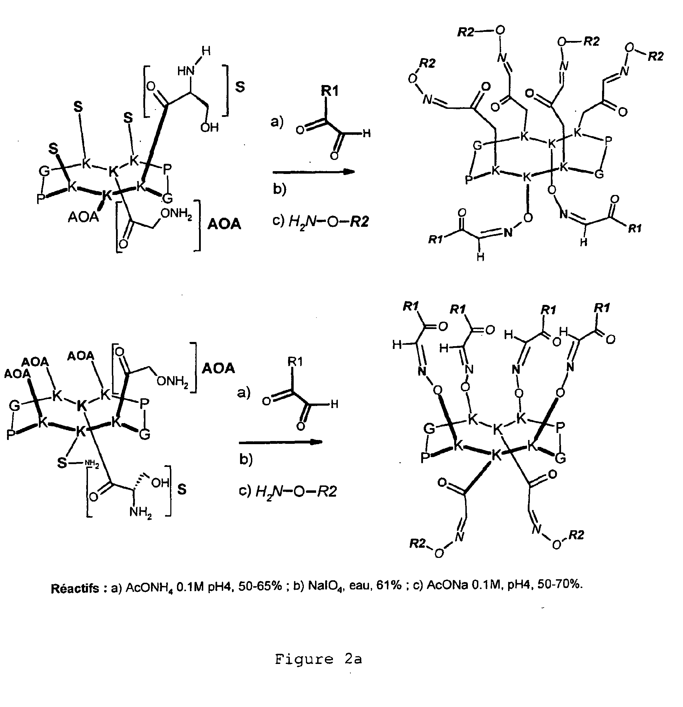

Les figures 2a et 2b illustrent le schéma de préparation de macromolécules polyfonctionnelles par assemblage chimiosélectif successif des biomolécules R1 et R2 par lien oxime, d'une part sur la face inférieure puis supérieure du cyclodécapeptide, et d'autre part sur la face supérieure puis inférieure du cyclodécapeptide.

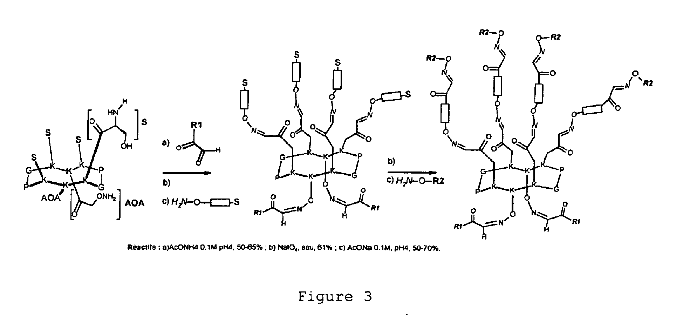

La figure 3 illustre le schéma de synthèse de macromolécules polyfonctionnelles par assemblage chimiosélectif successif par lien oxime, où la biomolécule R1 est greffée sur une face du cyclodécapeptide, où quatre molécules comportant une fonction oxyamine et au moins une sérine précurseur de la fonction α-oxoaldéhyde est greffée sur l'autre face du cyclodécapeptide, et finalement où la biomolécule R2 est greffée après démasquage des fonctions α-oxoaldéhydes.

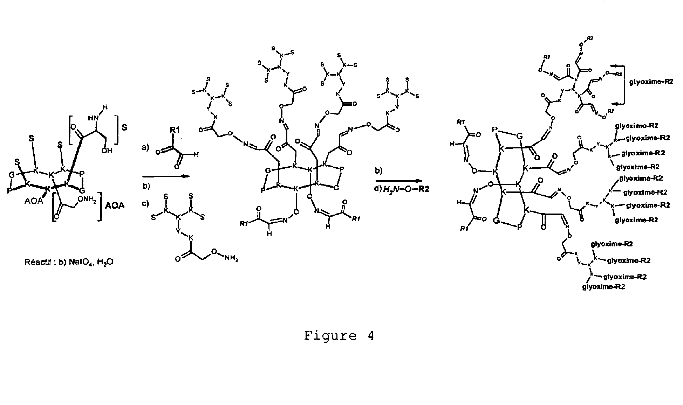

La figure 4 illustre le schéma de synthèse de macromolécules polyfonctionnelles par assemblage chimiosélectif successif par lien oxime, où la biomolécule R1 est greffée sur une face du cyclodécapeptide, où quatre molécules comportant une fonction oxyamine et quatre sérine précurseurs de la fonction α-oxoaldéhyde est greffée sur l'autre face du cyclodécapeptide, et finalement où la biomolécule R2 est greffée après démasquage des fonctions α-oxoaldéhydes.

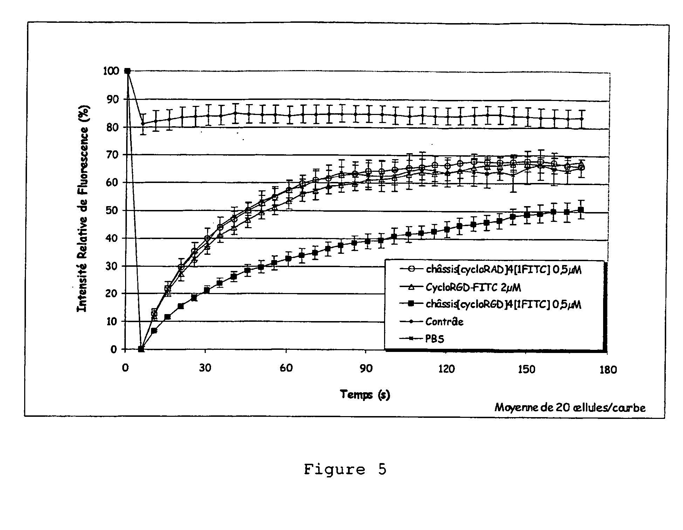

La figure 5 représente la récupération du signal de la fluorescence du récepteur αVβ3 après immunomarquage (anticorps LM609-R-**Phycoerythrine**) en fonction du temps pour les différentes conditions d'incubation des cellules vivantes HEK (expérience de FRAP) Le retard observé avec le composé multivalent RGD (carrés pleins) traduit une diminution de la mobilité des récepteurs αVβ3 caractéristique du phénomène de clustering non observé dans les autres conditions.

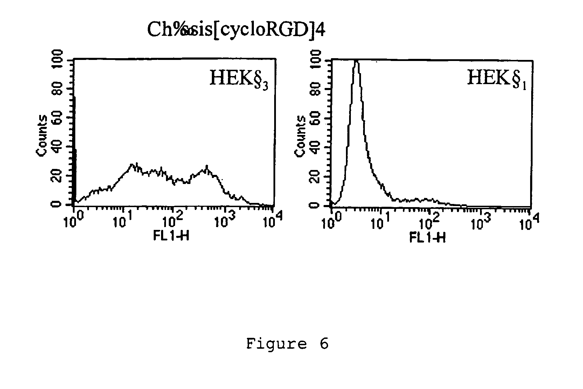

La figure 6 représente les histogrammes de fluorescence obtenus par FACS après incubation des cellules (HEK) avec le composé multivalent RGD-fluoresceine.

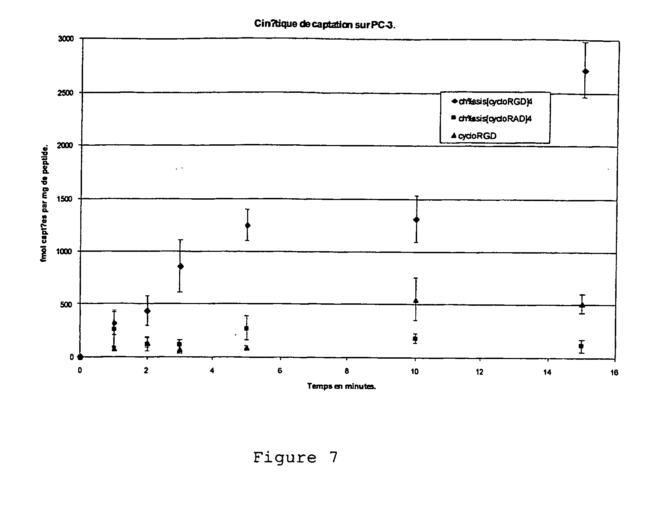

La figure 7 compare les cinétiques de captation par les cellules cancéreuse PC3 du composé multivalent RGD assemblé selon l'invention (RAFT[cycloRGD]₄ : losanges) à celles des molécules contrôles par mesure de l'activité radioactive de l'iode 125.

Selon le premier mode de réalisation illustré sur la figure 1a, on effectue en premier lieu la substitution des groupes protecteurs orthogonaux PG de la face inférieure soit PG2 et PG5, par un précurseur protégé de la fonction α-oxoaldéhyde, et de préférence Boc-Ser(tBu)OH ; dans une deuxième étape, on substitue les groupes protecteurs orthogonaux de la face supérieure du châssis, PG1, PG3, PG4, PG6 par un précurseur protégé de la fonction oxyamine, et de préférence Boc-AOA-OSu; dans une troisième étape représentée sur la figure 2a, on enlève tous les groupes protecteurs, la fonction oxyamine sur la face inférieure ainsi déprotégée va réagir en présence d'une première molécule d'intérêt portant une fonction α-oxoaldéhyde ; dans une quatrième étape représentée sur la figure 2a, on oxyde les résidus séryles, en fonction α-oxoaldéhyde sur la face supérieure ; dans une cinquième étape, on fait réagir les fonctions α-oxoaldéhydes de la face supérieure avec une seconde molécule d'intérêt portant une fonction oxyamine.

Selon le second mode de réalisation représenté sur les figures 1a et 2a, on inverse l'ordre de substitution des groupes protecteurs orthogonaux et greffage des faces supérieure et inférieure par rapport au mode premier respectivement. Suivant un mode de réalisation particulier de l'invention présenté figure 3, la dernière molécule d'intérêt greffée sur le châssis via sa fonction oxyamine permettant son greffage sur la ou les fonctions α-oxoaldéhydes situées sur le châssis, porte au moins un précurseur d'une fonction α-oxoaldéhyde qui permet de poursuivre le procédé selon l'invention en oxydant ce précurseur en α-oxoaldéhyde et en faisant réagir cette dernière avec une molécule d'intérêt présentant une fonction oxyamine, de manière à créer un lien oxime supplémentaire. Cette étape complémentaire permet la construction de système modulaire via couplage oxime itératif sur le châssis par démasquage de fonction α-oxoaldéhyde par oxydation successive.

Comme le décrit la figure 4, ce mode particulier permet avantageusement d'amplifier le nombre de molécules présentées in situ par le châssis dans le cas de molécule d'intérêt qui, greffée sur le châssis via sa fonction oxyamine permettant son greffage sur la ou les fonctions α-oxoaldéhydes situées sur le châssis, porte plus d'un précurseur d'une fonction α-oxoaldéhyde.

### Exemple 1 : cyclopeptide selon l'invention formant un châssis greffé sur une face avec des peptides dérivés du cyclo(RGDfK) et/ou du cyclo(RGDyK), qui sont ligands de l'intégrine αVβ3,

Les peptides cyclo(RGDfK) reconnaissent l'intégrine αVβ3. Cette intégrine est sur-exprimée à la surface cellulaire de tumeurs ou de cellules endothéliales lors de la néoangiogénèse tumorale. Le greffage de ce peptide sur une face du châssis selon l'invention, exalte les propriétés de reconnaissance de ce peptide par cette intégrine. De plus, les effets biologiques de cette intégrine, comme par exemple le clustering, et l'endocytose de la molécule, sont déclenchés par le châssis selon l'invention ainsi greffé, alors qu'ils ne sont pas déclenchés par le peptide seul.
1) **Support pour la culture de cellules in vitro :** Les châssis comportant un groupe biotine sur une de leur face permettent d'une manière générale la fonctionnalisation de cette face par de la steptavidine grâce à l'interaction forte biotine-streptavidine. L'autre face du châssis peut être fonctionnalisée par une molécule d'intérêt ou libre.
   L'utilisation de châssis comprenant par exemple des ligands d'adhésion comme le cyclo (RGDfK) permet la fixation de cellules exprimant le récepteur αVβ3 et leur culture *in vitro.* A basse densité de molécules adsorbées sur la surface, les composés se révèlent particulièrement efficaces.
2) **Induction d'un regroupement (clustering) de récepteurs cellulaires (figure 5) :** le regroupement des intégrines en surface des cellules HEK293 surexprimant l'intégrine β3 à été démontré par des expériences de FRAP. Les cellules vivantes sont mises en contact avec un anticorps anti αᵥβ₃ marqué à la Phycoerythrine (LM609-PE) et avec les différents peptides. Une zone de la surface cellulaire est alors photo-blanchie afin de pourvoir mesurer la cinétique de réapparition du signal fluorescent dans cette zone. Cette mesure reflète la vitesse de migration de l'intégrine αᵥβ₃ au sein de la membrane plasmique d'une cellule vivante. Si plusieurs molécules d'intégrines sont pontées par le peptide, alors le « cluster » ainsi formé migrera moins vite que l'intégrine αᵥβ₃ native, plus petite et plus libre. Les résultats présentés dans la figure 5 démontrent que seul le peptide obtenu selon l'invention est capable d'induire un tel regroupement d'intégrine αᵥβ₃ à la surface des cellules.
3) **Internalisation active des drogues ou produits vectorisés :** Les mêmes cellules mise en contact à 37 ou a 4°C pendant 15 à 30 minutes avec les peptides préalablement marqués à la fluoresceine sont observées en microscopie confocale après immunomarquage des vésicules d'endosomes précoces (marquage anti-Early Endosome 1A, visualisé en rouge).
   Les résultats obtenus démontrent que seul le peptide obtenu selon l'invention est visualisable à 37°C en surface, aux jonctions intercellulaires et à l'intérieur des cellules. Une partie importante du signal intracellulaire est co-localisée avec le marquage EE1A ce qui démontre qu'une partie au moins du peptide RAFT-RGD est internalisé par endocytose. Les peptides cRGD et RAFT-RGD incubés dans les mêmes conditions ne sont pas détectables par immunofluorescence. Ceci démontre leur incapacité à se fixer aussi efficacement que le composé obtenu selon l'invention sur leur intégrine cible. Si l'incubation des peptides est réalisée à 4°C, seul le marquage extracellulaire est observé avec le peptide obtenu selon l'invention, confirmant que l'internalisation à lieu grâce à un processus actif.
4) Vecteur ciblant pour le transfert de biomolécules (ADN, peptides, protéines, PNA, oligonucléotides, siRNA) :
   Seules les cellules exprimant l'intégrine αᵥβ₃ sont reconnues par le peptide obtenu selon l'invention. L'incubation de cellules HEK293 surexprimant soit l'intégrine β1 soit β3 avec le peptide fluorescent obtenu selon l'invention démontre que le peptide se fixe uniquement sur les cellules surexprimant l'hétérodimère αᵥβ₃. Cette propriété peut être utilisée selon l'invention pour le transport de molécules d'intérêt vers un récepteur cible.
5) **Tumeurs exprimant l'intégrine αvβ3** : Le peptide fluorescent obtenu selon l'invention injecté par voie intraveineuse chez une souris porteuse de tumeurs révèle à l'imagerie médicale que ce peptide est retrouvé fortement accumulé dans les zones hypervascularisées de la tumeur.
6) Vaisseaux sanguins
   Nos résultats obtenus avec les peptides fluorescents obtenus selon l'invention démontrent que l'endothélium peut être reconnu par ces molécules injectées par voie intraveineuse. D'autres tissus et notamment la capsule hépatique, les vaisseaux du foie et la rate ont aussi été imagés grâce à ces molécules. Le greffage d'autres ligands devrait permettre d'augmenter le signal obtenu sur l'endothélium.
7) Activités enzymatiques en médecine ou in vitro sur culture de cellules (activité protéasiques notamment)
   Selon l'invention, il est possible de greffer sur le châssis des peptides contenant une séquence spécifiquement reconnue par une protéase. Si l'on greffe une molécule fluorescente en amont du site de clivage et une molécule qui absorbe la fluorescence en aval de ce site, la faible distance entre les 2 marqueurs empêche toute émission de fluorescence. Par contre, en présence de la protéase spécifique, les 2 fluorophores sont dissociés, et il y a donc émission de lumière quantifiable. Cette mesure permet de déterminer la concentration en protéase présente dans le compartiment, tissu ou cellule observé. L'invention permet de diriger vers des tissus cibles ce système afin d'imager spécifiquement ces régions.
8) **Radiodétection des tissus endotheliaux lors de la néoangiogénèse tumorale ou des tumeurs exprimant l'intégrine _V_3. (figure 7)**
   Le marquage par l'iode 127 radioactif permet la détection de la molécules et donc de sa liaison à l'intégrine à la surface cellulaire puis de son entrée dans le cytosol après endocytose. L'effet de captation est dose dépendant en composés. Cet effet a été mesuré sur des cellules tumorales (PC-3) et endothéliales (HMVEC)exprimant l'intégrine. Ici encore seuls les cyclopeptides greffés selon l'invention possèdent l'effet observé contrairement au ligand isolé. Cette propriété permet l'imagerie des zones de néoangigénèse par mesure de l'activité radioactive.

### Exemple 2 : cyclopeptide selon l'invention formant un châssis greffé sur une face avec des épitopes B-dépendant de type carbohydrate plus particulièrement marqueur de tumeur (Tn, sTn, Tf), un ou des épitopes T-dépendants (peptides Thl ou Th2) et un immunoadjuvant.

Ces substituants sont choisis pour susciter une réponse cellulaire, dans un but de vaccination.

Les sucres sont connus pour être des éléments très importants dans de nombreuses pathologies notamment le cancer (marqueur de tumeur) ou les infections virales et bactériennes. Cependant ils sont faiblement immunogène ce qui limitent grandement leur utilisation dans des compositions vaccinales à visées thérapeutiques évidentes. De plus la reconnaissance de motifs sucres passent par une présentation sous forme de grappes et est également une limite à leur utilisation. L'invention permet la présentation de motifs sucre sous forme de grappes et également leur manipulation chimique pour réaliser des combinaisons épitopiques permettant à terme de susciter un apprentissage du système immunitaire pour protéger l'organisme des pathologies et infections impliquant ces motifs.

## Revendications

1. Procédé de préparation d'un cyclopeptide homodétique greffé formant un châssis définissant deux faces, une face dite supérieure et une face dite inférieure, lesdites deux faces étant toutes les deux greffées, **caractérisé en ce que** l'on synthétise un peptide linéaire, ladite synthèse étant effectuée à partir d'acides aminés modifiés ou non, certains d'entre eux portant des groupes protecteurs orthogonaux, on effectue une cyclisation intramoléculaire du peptide protégé linéaire obtenu, on substitue tout ou partie des groupes protecteurs orthogonaux par un précurseur protégé, on greffe sur l'une et/ou l'autre face dudit châssis, par une liaison oxime, au moins une molécule d'intérêt.

2. Procédé de préparation d'un cyclopeptide homodétique greffé selon la revendication 1, **caractérisé en ce que** ladite synthèse du peptide linéaire, effectuée sur phase solide, est initiée à partir d'un résidu de glycine dont la fonction carboxylique est ancrée à une résine, et la cyclisation du peptide linéaire obtenu est effectuée en solution après libération de la résine.

3. Procédé de préparation d'un cyclopeptide homodétique greffé selon la revendication 1, **caractérisé en ce que** ladite synthèse du peptide linéaire puis sa cyclisation sont entièrement effectuées sur phase solide.

4. Procédé de préparation d'un cyclopeptide homodétique greffé selon la revendication 3, **caractérisé en ce que** ladite synthèse du peptide linéaire est initiée par un résidu d'acide aminé dont la chaîne latérale est ancrée à une résine.

5. Procédé de préparation d'un cyclopeptide homodétique greffé selon l'une quelconque des revendications 1 à 4, **caractérisé en ce qu'**il est entièrement ou partiellement automatisé sur robot synthétiseur de peptides.

6. Procédé de préparation d'un cyclopeptide homodétique greffé selon l'une quelconque des revendications 1 à 5, **caractérisé en ce que** ledit cyclopeptide est formé de 5, 10 ou 14 résidus d'acides aminés, de préférence 10 acides aminés formant un cyclodécapeptide.

7. Procédé de préparation d'un cyclopeptide homodétique greffé selon la revendication 5, **caractérisé en ce que** le cyclopeptide présente 10 ou 14 résidus d'acides aminés et forme deux coudes, lesdits deux coudes étant formés par une combinaison (L)Pro-(D)AA ou/et (D)Pro-(L)AA, AA étant un acide aminé et de préférence la glycine, les deux coudes étant séparés par trois ou cinq résidus d'acides aminés respectivement.

8. Procédé de préparation d'un cyclopeptide homodétique greffé selon l'une quelconque des revendications 6 ou 7, **caractérisé en ce que** lesdits trois ou cinq résidus d'acides aminés ont chacun, sur leur chaîne latérale, une fonction chimique initialement protégée orthogonalement par un groupe protecteur, les groupes protecteurs des chaînes latérales de ces acides aminés se dirigent alternativement de part et d'autre du plan médian dudit châssis et définissent une face dite inférieure et supérieure par rapport à ce plan.

9. Procédé de préparation d'un cyclopeptide homodétique greffé selon l'une quelconque des revendications 6 à 8, **caractérisé en ce** lesdits trois ou cinq résidus d'acides aminés sont préférentiellement des résidus aminoacides à chaîne latérale amine, et très préférentiellement la lysine.

10. Procédé de préparation d'un cyclopeptide homodétique greffé selon l'une quelconque des revendications 6 à 9, **caractérisé en ce que** les groupes protecteurs orthogonaux desdits résidus d'acides aminés centraux sont identiques entre eux, les groupes protecteurs orthogonaux desdits autres résidus d'acides aminés sont identiques entre eux, les groupes protecteurs orthogonaux desdits résidus d'acides aminés centraux d'une part, et les groupes protecteurs orthogonaux desdits autres résidus d'acides aminés d'autre part, sont différents les uns des autres.

11. Procédé de préparation d'un cyclopeptide homodétique greffé selon la revendication 1, **caractérisé en ce que** l'on débute le greffage du châssis en substituant des groupes protecteurs orthogonaux par un précurseur protégé de la fonction oxyamine ou un précurseur masqué protégé de la fonction aldéhyde ou par un marqueur.

12. Procédé de préparation d'un cyclopeptide homodétique greffé selon la revendication 11, **caractérisé en ce que** ledit précurseur protégé est l'acide 2-oxyamino-acétique protégé (AOA).

13. Procédé de préparation d'un cyclopeptide homodétique greffé selon la revendication 11, **caractérisé en ce que** ledit précurseur masqué protégé est un résidu de la sérine dont les fonctions amine et hydroxyle sont protégées, et dont l'oxydation libère un groupement aldéhyde, et de préférence est Boc-Ser (tBu) OH.

14. Procédé de préparation d'un cyclopeptide homodétique greffé selon la revendication 11, **caractérisé en ce que** ledit précurseur protégé est précurseur de la fonction thiol, de préférence un dérivé disulfure disymétrique de la cystéine et de manière très préférentielle un groupe Npys.

15. Procédé de préparation d'un cyclopeptide homodétique greffé selon l'une quelconque des revendications 11 à 14, **caractérisé en ce que** l'on effectue en premier lieu la substitution des groupes protecteurs orthogonaux de la face inférieure par un marqueur, de préférence la biotine ou la fluorescéine, puis dans une deuxième étape, on substitue les groupes protecteurs orthogonaux de la face supérieure du châssis par un précurseur protégé de la fonction oxyamine ou de la fonction aldéhyde.

16. Procédé de préparation d'un cyclopeptide homodétique greffé selon l'une quelconque des revendications 11 à 14, **caractérisé en ce que** l'on effectue en premier lieu la substitution des groupes protecteurs orthogonaux de la face inférieure du châssis par un précurseur protégé de la fonction oxyamine puis dans une deuxième étape on substitue les groupes protecteurs orthogonaux de la face supérieure du cyclopeptide par un précurseur masqué protégé de la fonction aldéhyde.

17. Procédé de préparation d'un cyclopeptide homodétique greffé selon l'une quelconque des revendications 11 à 14, **caractérisé en ce que** l'on effectue en premier lieu la substitution des groupes protecteurs orthogonaux de la face supérieure du châssis par un précurseur protégé de la fonction oxyamine puis dans une deuxième étape on substitue les groupes protecteurs orthogonaux de la face inférieure du cyclopeptide par un précurseur masqué protégé de la fonction aldéhyde.

18. Procédé de préparation d'un cyclopeptide homodétique greffé selon l'une quelconque des revendications 11 à 17, **caractérisé en ce que** l'on fait réagir les fonctions oxyamine ou aldéhyde générées à partir des précurseurs, préalablement déprotégées, avec une ou plusieurs molécules d'intérêt ou une molécule intermédiaire portant une fonction respectivement aldéhyde ou oxyamine.

19. Procédé de préparation d'un cyclopeptide homodétique greffé selon la revendication 18, **caractérisé en ce que** lesdites molécules d'intérêt sont identiques ou différentes les unes des autres.

20. Procédé de préparation d'un cyclopeptide homodétique greffé selon l'une quelconque des revendications 18 ou 19, **caractérisé en ce que** lesdites molécules d'intérêts sont des acides nucléiques, des peptides, des oligosaccharides, ou des molécules organiques.

21. Procédé de préparation d'un cyclopeptide homodétique greffé selon la revendication 20, **caractérisé en ce que** l'une au moins des molécules d'intérêt est le cyclopentapeptide c(RGDfK).

22. Procédé de préparation d'un cyclopeptide homodétique greffé selon l'une quelconque des revendications 18 à 21, **caractérisé en ce que** l'on fait réagir la fonction oxyamine du précurseur situé sur le châssis avec au moins une molécule d'intérêt portant une fonction aldéhyde, puis on oxyde le précurseur de la fonction aldéhyde situé sur le châssis et l'on poursuit la réaction avec une mise en contact du châssis avec une molécule d'intérêt ou une molécule intermédiaire portant une fonction oxyamine.

23. Procédé de préparation d'un cyclopeptide homodétique greffé selon l'une quelconque des revendications 18 à 22, **caractérisé en ce que** ladite molécule intermédiaire porte d'une part une fonction oxyamine capable de réagir avec la ou les fonctions aldéhydes situées sur le châssis, et porte d'autre part un précurseur d'au moins une fonction aldéhyde.

24. Procédé de préparation d'un cyclopeptide homodétique greffé selon l'une quelconque des revendications 3 à 23, **caractérisé en ce qu'**il est entièrement ou partiellement automatisé sur robot synthétiseur de peptide.

25. Cyclopeptide homodétique greffé **caractérisé en ce qu'**il est susceptible d'être obtenu par le procédé selon l'une quelconque des revendications 1 à 24.

26. Cyclopeptide homodétique greffé selon la revendication 25, **caractérisé en ce qu'**il est greffé sur une de ses faces par un ligand de l'intégrine αVβ3, de préférence des peptides dérivés du cyclo(RGDfK) et/ou du cyclo(RGDyK), qui sont ligands de l'intégrine, et sur l'autre de ses faces par un peptide apoptogène du type KLAKKLAK, une molécule organique thérapeutique connue du type doxorobucine, ou une protéine toxique au niveau intracellulaire.

27. Cyclopeptide homodétique greffé selon la revendication 25, **caractérisé en ce qu'**il est greffé sur une de ses faces par un ligand de l'intégrine αVβ3, de préférence des peptides dérivés du cyclo(RGDfK) et/ou du cyclo(RGDyK), qui sont ligands de l'intégrine, et sur l'autre de ses faces par une molécule détectable, du type chromophore(s), biotine(s), fluorophore(s), radio-émetteur(s) ou un précurseur.

28. Cyclopeptide homodétique greffé selon la revendication 25, **caractérisé en ce qu'**il est greffé sur une de ses faces avec des dérivés carbohydrates et sur l'autre face avec un ou des peptides(s) épitope(s) T-dépendant, un ou des peptides cytotoxiques, une ou des molécules organiques thérapeutiques ou une protéine toxique au niveau intracellulaire.

29. Cyclopeptide homodétique greffé selon la revendication 25, **caractérisé en ce qu'**il est greffé sur une de ses faces avec des dérivés carbohydrates et sur l'autre face du châssis avec un ou des chromophore(s), une ou des biotine(s) ; un ou des fluorophore(s), une ou des radio-émetteur(s) ou un groupe précurseur chimique ou ligand.

30. Cyclopeptide homodétique greffé selon la revendication 25, **caractérisé en ce qu'**il est greffé sur une face avec des épitopes B-dépendant de type carbohydrate ou des épitopes T-dépendants et un immunoadjuvant.

31. Composition thérapeutique ou de diagnostic **caractérisé en ce qu'**elle comprend un cyclopeptide homodétique greffé selon la revendication 25.

32. Utilisation d'un cyclopeptide selon la revendication 25 ou d'une composition selon la revendication 31, pour la fabrication d'un médicament destiné à soigner le cancer.

33. Utilisation d'un cyclopeptide selon la revendication 25 ou d'une composition selon la revendication 31, pour la fabrication d'un outil de diagnostic du cancer.

34. Utilisation d'un cyclopeptide selon la revendication 25 ou d'une composition selon la revendication 31, pour le diagnostic de la néoangiogénèse.

35. Utilisation d'un cyclopeptide selon la revendication 25 ou d'une composition selon la revendication 31, pour la suppression de la néoangiogénèse.

## Claims

1. Method for preparing a grafted homodetic cyclopeptide forming a framework that defines two faces, a so-called upper face and a so-called lower face, said two faces both being grafted, **characterized in that** a linear peptide is synthesized, said synthesis being performed from modified or unmodified amino acids, some of which carry orthogonal protective groups; an intramolecular cyclization of the resulting protected linear peptide is performed, some or all of the orthogonal protective groups are substituted with a protected precursor, at least one molecule of interest is grafted onto one and/or the other face of said framework via an oxime bond.

2. Method for preparing a grafted homodetic cyclopeptide according to Claim 1, **characterized in that** said synthesis of the linear peptide, performed on the solid phase, is initiated from a glycine residue whose carboxyl function is anchored to a resin, and cyclization of the resulting linear peptide is performed in solution after release of the resin.

3. Method for preparing a grafted homodetic cyclopeptide according to Claim 1, **characterized in that** said synthesis of the linear peptide and then cyclization thereof are performed entirely on the solid phase.

4. Method for preparing a grafted homodetic cyclopeptide according to Claim 3, **characterized in that** said synthesis of the linear peptide is initiated with an amino acid residue whose side chain is anchored to a resin.

5. Method for preparing a grafted homodetic cyclopeptide according to any of Claims 1 to 4, **characterized in that** it is entirely or partially automated on a peptide-synthesizing robot.

6. Method for preparing a grafted homodetic cyclopeptide according to any of Claims 1 to 5, **characterized in that** said cyclopeptide is constituted from 5, 10, or 14 amino acid residues, preferably 10 amino acids forming a cyclodecapeptide.

7. Method for preparing a grafted homodetic cyclopeptide according to Claim 5, **characterized in that** the cyclopeptide exhibits 10 or 14 amino acid residues and forms two turns, said two turns being constituted by an (L)Pro-(D)AA and/or (D)Pro-(L)AA combination, AA being an amino acid and preferably glycine, the two turns being separated by three or five amino acid residues, respectively.

8. Method for preparing a grafted homodetic cyclopeptide according toeither of Claims 6 or 7, **characterized in that** said three or five amino acid residues each have, on their side chain, a chemical function initially protected orthogonally by a protective group, the protective groups of the side chains of these amino acids being directed alternately to one side and the other of the median plane of said framework, and defining a so-called lower and upper face with respect to that plane.

9. Method for preparing a grafted homodetic cyclopeptide according toany of Claims 6 to 8, **characterized in that** said three or five amino acid residues are preferably amino acid residues having an amine side chain, and very preferably lysine.

10. Method for preparing a grafted homodetic cyclopeptide according toany of Claims 6 to 9, **characterized in that** the orthogonal protective groups of said central amino acid residues are identical to one another, the orthogonal protective groups of said other amino acid residues are identical to one another, the orthogonal protective groups of said central amino acid residues, on the one hand, and the orthogonal protective groups of said other amino acid residues, on the other hand, are different from one another.

11. Method for preparing a grafted homodetic cyclopeptide according to Claim 1, **characterized in that** grafting of the framework is begun by substituting the orthogonal protective groups of the framework with a protected precursor of the oxyamine function or a protected masked precursor of the aldehyde function, or with a label.

12. Method for preparing a grafted homodetic cyclopeptide according to Claim 11, **characterized in that** said protected precursor is protected 2-oxyaminoacetic acid (OAA).

13. Method for preparing a grafted homodetic cyclopeptide according toClaim 11, **characterized in that** said protected masked precursor is a serine residue, the amine and hydroxyl functions of which are protected, and oxidation of which releases an aldehyde group, and preferably is Boc-Ser(tBu)OH.

14. Method for preparing a grafted homodetic cyclopeptide according to Claim 11, **characterized in that** said protected precursor is a precursor of the thiol function, preferably a dissymmetrical disulfide derivative of cysteine, and very preferably is an Npys group.

15. Method for preparing a grafted homodetic cyclopeptide according to any of Claims 11 to 14, **characterized in that** firstly substitution of the orthogonal protective groups of the lower face with a label, preferably biotin or fluorescein, is performed; then in a second step the orthogonal protective groups of the upper face of the framework are substituted with a protected precursor of the oxyamine function or of the aldehyde function.

16. Method for preparing a grafted homodetic cyclopeptide according to any of Claims 11 to 14, **characterized in that** firstly substitution of the orthogonal protective groups of the lower face of the framework with a protected precursor of the oxyamine function is performed; then, in a second step, the orthogonal protective groups of the upper face of the cyclopeptide are substituted with a protected masked precursor of the aldehyde function.

17. Method for preparing a grafted homodetic cyclopeptide according to any of Claims 11 to 14, **characterized in that** firstly substitution of the orthogonal protective groups of the upper face of the framework with a protected precursor of the oxyamine function is performed; then, in a second step, the orthogonal protective groups of the lower face of the cyclopeptide are substituted with a protected masked precursor of the aldehyde function.

18. Method for preparing a grafted homodetic cyclopeptide according to any of Claims 11 to 17, **characterized in that** the oxyamine or aldehyde functions generated from the precursors, previously deprotected, are reacted with one or several molecules of interest or with an intermediate molecule carrying an aldehyde or oxyamine function, respectively.

19. Method for preparing a grafted homodetic cyclopeptide according to Claim 18, **characterized in that** said molecules of interest are identical to or different from one another.

20. Method for preparing a grafted homodetic cyclopeptide according to either of Claims 18 or 19, **characterized in that** said molecules of interest are nucleic acids, peptides, oligosaccharides, or organic molecules.

21. Method for preparing a grafted homodetic cyclopeptide according to Claim 20, **characterized in that** at least one of the molecules of interest is the cyclopentapeptide c(RGDfK).

22. Method for preparing a grafted homodetic cyclopeptide according to any of Claims 18 to 21, **characterized in that** the oxyamine function of the precursor located on the framework is reacted with at least one molecule of interest carrying an aldehyde function, then the precursor of the aldehyde function located on the framework is oxidized and the reaction is continued by bringing the framework into contact with a molecule of interest or an intermediate molecule carrying an oxyamine function.

23. Method for preparing a grafted homodetic cyclopeptide according to any of Claims 18 to 22, **characterized in that** said intermediate molecule on the one hand carries an oxyamine function capable of reacting with the aldehyde function(s) located on the framework, and on the other hand carries a precursor of at least one aldehyde function.

24. Method for preparing a grafted homodetic cyclopeptide according to any of Claims 3 to 23, **characterized in that** it is entirely or partially automated on a peptide-synthesizing robot.

25. A grafted homodetic cyclopeptide, **characterized in that** it is obtainable by the method according to any of Claims 1 to 24.

26. The grafted homodetic cyclopeptide according to Claim 25, **characterized in that** it is grafted on one of its faces with a ligand of integrin αvβ3, preferably peptides derived from cyclo(RGDfK) and/or cyclo(RGDyK), which are ligands of integrin, and on the other of its faces with an apoptogenic peptide of the KLAKKLAK type, a known therapeutic organic molecule of the doxorobucin type, or a protein that is toxic at the intracellular level.

27. The grafted homodetic cyclopeptide according to Claim 25, **characterized in that** it is grafted on one of its faces with a ligand of integrin αvβ3, preferably peptides derived from cyclo(RGDfK) and/or cyclo(RGDyK), which are ligands of integrin, and on the other of its faces with a detectable molecule of the chromophore, biotin, fluorophore, radioemitter type, or a precursor.

28. The grafted homodetic cyclopeptide according to Claim 25, **characterized in that** it is grafted on one of its faces with carbohydrate derivatives and on the other face with one or several T-dependent epitopic peptides, one or several cytotoxic peptides, one or several therapeutic organic molecule(s), or a protein that is toxic at the intracellular level.

29. The grafted homodetic cyclopeptide according to Claim 25, **characterized in that** it is grafted on one of its faces with carbohydrate derivatives and on the other face of the framework with one or several chromophore(s), one or several biotin(s), one or several fluorophore(s), one or several radioemitter(s), or a chemical precursor group or ligand.

30. The grafted homodetic cyclopeptide according to Claim 25, **characterized in that** it is grafted on one face with B-dependent epitopes of the carbohydrate type, or T-dependent epitopes, and an immunoadjuvant.

31. A therapeutic or diagnostic composition, **characterized in that** it comprises a grafted homodetic cyclopeptide according to Claim 25.

32. Use of a cyclopeptide according to Claim 25, or a composition according to Claim 31, for production of a medication intended to treat cancer.

33. Use of a cyclopeptide according to Claim 25, or a composition according to Claim 31, for production of a tool for diagnosing cancer.

34. Use of a cyclopeptide according to Claim 25, or a composition according to Claim 31, for the diagnosis of neoangiogenesis.

35. Use of a cyclopeptide according to Claim 25, or a composition according to Claim 31, for the suppression of neoangiogenesis.

## Patentansprüche

1. Verfahren zur Herstellung eines gepfropften homodetischen Cyclopeptids, ein zwei Seiten definierendes Chassis bildend, eine obere Seite und eine untere Seite genannt, wobei die beiden Seiten gepfropft sind, **dadurch gekennzeichnet, dass** man ein lineares Peptid synthetisiert, wobei die besagte Synthese ausgehend von modifizierten oder nicht modifizierten Aminosäuren durchgeführt wird, wobei einige von ihnen orthogonale Schutzgruppen tragen, man eine intramolekulare Cyclisierung des hergestellten linearen geschützten Proteins durchführt, alle oder einen Teil der orthogonalen Schutzgruppen durch eine geschützte Vorstufe ersetzt, man auf die eine und/oder andere Seite des besagten Chassis mittels einer Oximverbindung mindestens ein interessierendes Molekül pfropft.

2. Verfahren zur Herstellung eines gepfropften homodetischen Cyclopeptids nach Anspruch 1, **dadurch gekennzeichnet, dass** die besagte, auf der soliden Phase durchgeführte Synthese des linearen Peptids ausgehend von einem Glycinrest initiiert wird, dessen Carboxylfunktion mit einem Harz verankert ist, und die Cyclisierung des hergestellten linearen Peptids wird in Lösung nach Freisetzung des Harzes durchgeführt.

3. Verfahren zur Herstellung eines gepfropften homodetischen Cyclopeptids nach Anspruch 1, **dadurch gekennzeichnet, dass** die besagte Synthese des linearen Peptids und danach seine Cyclisierung voll und ganz auf der soliden Phase durchgeführt werden.

4. Verfahren zur Herstellung eines gepfropften homodetischen Cyclopeptids nach Anspruch 3, **dadurch gekennzeichnet, dass** die besagte Synthese des linearen Peptids von einem Aminosäurerest initiiert wird, dessen Seitenkette mit einem Harz verankert ist.

5. Verfahren zur Herstellung eines gepfropften homodetischen Cyclopeptids nach einem der Ansprüche 1 bis 4, **dadurch gekennzeichnet, dass** es voll und ganz auf Peptidsynthese-Roboter automatisiert ist.

6. Verfahren zur Herstellung eines gepfropften homodetischen Cyclopeptids nach einem der Ansprüche 1 bis 5, **dadurch gekennzeichnet, dass** das besagte Cyclopeptid von 5, 10 oder 14 Aminosäureresten gebildet wird, vorzugsweise von 10 Aminosäuren, die ein Cyclodecapeptid bilden.

7. Verfahren zur Herstellung eines gepfropften homodetischen Cyclopeptids nach Anspruch 5, **dadurch gekennzeichnet, dass** das Cyclopeptid 10 oder 14 Aminosäurereste aufweist und zwei Krümmungen bildet, wobei die besagten zwei Krümmungen von einer Kombination (L)Pro-(D)AA oder/und (D)Pro-(L)AA gebildet werden, wobei AA eine Aminosäure und vorzugsweise Glycin ist, wobei die zwei Krümmungen jeweils von drei oder fünf Aminosäureresten separiert werden.

8. Verfahren zur Herstellung eines gepfropften homodetischen Cyclopeptids nach einem der Ansprüche 6 oder 7, **dadurch gekennzeichnet, dass** die besagten drei oder fünf Aminosäurereste jeweils auf ihrer Seitenkette eine ursprünglich von einer Schutzgruppe orthogonal geschützte chemische Funktion haben, wobei sich die Schutzgruppen der Seitenketten dieser Aminosäuren abwechselnd auf beiden Seiten der Medianebene des besagten Chassis ausrichten und eine untere und obere Seite in Bezug zu dieser Ebene definieren.

9. Verfahren zur Herstellung eines gepfropften homodetischen Cyclopeptids nach einem der Ansprüche 6 bis 8, **dadurch gekennzeichnet, dass** die besagten drei oder fünf Aminosäurereste vorzugsweise Aminosäurereste mit seitlicher Aminkette und sehr vorzugsweise Lysin sind.

10. Verfahren zur Herstellung eines gepfropften homodetischen Cyclopeptids nach einem der Ansprüche 6 bis 9, **dadurch gekennzeichnet, dass** die orthogonalen Schutzgruppen der besagten zentralen Aminosäurereste untereinander identisch sind, wobei die orthogonalen Schutzgruppen der besagten anderen Aminosäurereste untereinander identisch sind, wobei die orthogonalen Schutzgruppen der besagten zentralen Aminosäurereste einerseits und die orthogonalen Schutzgruppen der besagten anderen Aminosäurereste andererseits voneinander unterschiedlich sind.

11. Verfahren zur Herstellung eines gepfropften homodetischen Cyclopeptids nach Anspruch 1, **dadurch gekennzeichnet, dass** man die Pfropfung des Chassis durch Substitution der orthogonalen Schutzgruppen durch eine geschützte Vorstufe der Oxiaminfunktion oder eine geschützte maskierte Vorstufe der Aldehydfunktion oder durch einen Marker beginnt.

12. Verfahren zur Herstellung eines gepfropften homodetischen Cyclopeptids nach Anspruch 11, **dadurch gekennzeichnet, dass** die besagte Vorstufe die geschützte 2-Aminooxy-Essigsäure (AOA) ist.

13. Verfahren zur Herstellung eines gepfropften homodetischen Cyclopeptids nach Anspruch 11, **dadurch gekennzeichnet, dass** die besagte geschützte maskierte Vorstufe ein Serinrest ist, dessen Amin- und Hydroxylfunktionen geschützt sind, und dessen Oxydation eine Aldehydgruppe freisetzt, und vorzugsweise Boc-Ser(tBu)OH ist.

14. Verfahren zur Herstellung eines gepfropften homodetischen Cyclopeptids nach Anspruch 11, **dadurch gekennzeichnet, dass** die besagte geschützte Vorstufe Vorstufe der Thiolfunktion ist, vorzugsweise ein disymmetrisches Disulfidderivat des Cysteins und sehr vorzugsweise eine Npys-Gruppe.

15. Verfahren zur Herstellung eines gepfropften homodetischen Cyclopeptids nach einem der Ansprüche 11 bis 14, **dadurch gekennzeichnet, dass** man zunächst die Substitution der orthogonalen Schutzgruppen der unteren Seite durch einen Marker, vorzugsweise Biotin oder Fluorescein, durchführt und danach in einem zweiten Schritt die orthogonalen Schutzgruppen der oberen Seite des Chassis durch eine geschützte Vorstufe der Oxiaminfunktion oder der Aldehydfunktion substituiert.

16. Verfahren zur Herstellung eines gepfropften homodetischen Cyclopeptids nach einem der Ansprüche 11 bis 14, **dadurch gekennzeichnet, dass** man zunächst die Substitution der orthogonalen Schutzgruppen der unteren Seite des Chassis durch eine geschützte Vorstufe der Oxiaminfunktion durchführt und danach in einem zweiten Schritt die orthogonalen Schutzgruppen der oberen Seite des Cyclopeptids durch eine geschützte maskierte Vorstufe der Aldehydfunktion substituiert.

17. Verfahren zur Herstellung eines gepfropften homodetischen Cyclopeptids nach einem der Ansprüche 11 bis 14, **dadurch gekennzeichnet, dass** man zunächst die Substitution der orthogonalen Schutzgruppen der oberen Seite des Chassis durch eine geschützte Vorstufe der Oxiaminfunktion durchführt und danach in einem zweiten Schritt die orthogonalen Schutzgruppen der unteren Seite des Cyclopeptids durch eine geschützte maskierte Vorstufe der Aldehydfunktion substituiert.

18. Verfahren zur Herstellung eines gepfropften homodetischen Cyclopeptids nach einem der Ansprüche 11 bis 17, **dadurch gekennzeichnet, dass** man die aus Vorstufen erzeugten Oxiamin- oder Aldehydfunktionen, deren Schutz zuvor aufgehoben wurde, mit einem oder mehreren interessierenden Molekülen oder einem intermediären Molekül, das eine Aldehy- bzw. Oxiaminfunktion trägt, zur Reaktion bringt.

19. Verfahren zur Herstellung eines gepfropften homodetischen Cyclopeptids nach Anspruch 18, **dadurch gekennzeichnet, dass** die besagten interessierenden Moleküle untereinander identisch sind oder sich voneinander unterscheiden.

20. Verfahren zur Herstellung eines gepfropften homodetischen Cyclopeptids nach einem der Ansprüche 18 oder 19, **dadurch gekennzeichnet, dass** die besagten interessierenden Moleküle Nucleinsäuren, Peptide, Oligosaccharide oder organische Moleküle sind.

21. Verfahren zur Herstellung eines gepfropften homodetischen Cyclopeptids nach Anspruch 20, **dadurch gekennzeichnet, dass** zumindest eines der interessierenden Moleküle das Cyclopentapeptid c (RGDfK) ist.

22. Verfahren zur Herstellung eines gepfropften homodetischen Cyclopeptids nach einem der Ansprüche 18 bis 21, **dadurch gekennzeichnet, dass** man die Oxiaminfunktion der auf dem Chassis befindlichen Vorstufe mit mindestens einem interessierenden Molekül, das eine Aldehydfunktion trägt, zur Reaktion bringt, man danach die auf dem Chassis befindliche Vorstufe der Aldehydfunktion oxidiert und man die Reaktion durch Kontaktaufnahme des Chassis mit einem interessierenden Molekül oder einem intermediären Molekül, das eine Oxiaminfunktion trägt, fortsetzt.

23. Verfahren zur Herstellung eines gepfropften homodetischen Cyclopeptids nach einem der Ansprüche 18 bis 22, **dadurch gekennzeichnet, dass** das besagte intermediäre Molekül einerseits eine Oxiaminfunktion trägt, die in der Lage ist, mit der oder den auf dem Chassis befindlichen Aldehydfunktionen zu reagieren, und andererseits eine Vorstufe mindestens einer Aldehydfunktion trägt.

24. Verfahren zur Herstellung eines gepfropften homodetischen Cyclopeptids nach einem der Ansprüche 3 bis 23, **dadurch gekennzeichnet, dass** es voll und ganz oder teilweise auf Peptidsynthese-Roboter automatisiert ist.

25. Gepfropftes homodetisches Cyclopeptid, **dadurch gekennzeichnet, dass** es gemäß dem Verfahren nach einem der Ansprüche 1 bis 24 hergestellt werden kann.

26. Gepfropftes homodetisches Cyclopeptid nach Anspruch 25, **dadurch gekennzeichnet, dass** es auf einer seiner Seiten durch einen Liganden des Integrins αVβ3 gepfropft wird, vorzugsweise von vom Cyclo(RGDfK) und/oder vom Cyclo(RGDyK) abgeleiteten Peptiden, die Liganden des Integrins sind, und auf seiner anderen Seite durch ein apoptogenes Peptid vom Typ KLAKKLAK, ein bekanntes therapeutisches organisches Molekül vom Doxorubicintyp oder ein auf intrazellulärer Ebene toxisches Protein.

27. Gepfropftes homodetisches Cyclopeptid nach Anspruch 25, **dadurch gekennzeichnet, dass** es auf einer seiner Seiten durch einen Liganden des Integrins αVβ3 gepfropft wird, vorzugsweise von vom Cyclo(RGDfK) und/oder vom Cyclo(RGDyK) abgeleiteten Peptiden, die Liganden des Integrins sind, und auf seiner anderen Seite durch ein ansprechbares Molekül vom Typ Chromophor(e), Biotin(e), Fluorophor(e), Funküberträger oder einer Vorstufe.

28. Gepfropftes homodetisches Cyclopeptid nach Anspruch 25, **dadurch gekennzeichnet, dass** es auf einer seiner Seiten mit Kohlenhydratderivaten und auf der anderen Seite mit einem T-abhängigen Epitopeptid oder Epitopeptiden, einem zytotoxischen Peptide oder Peptiden, einem therapeutischen organischen Molekül oder Molekülen oder einem auf intrazellulärer Ebene toxischen Protein gepfropft ist.

29. Gepfropftes homodetisches Cyclopeptid nach Anspruch 25, **dadurch gekennzeichnet, dass** es auf einer seiner Seiten mit Kohlenhydratderivaten und auf der anderen Seite des Chassis mit Chromophor oder Chromophoren, einem Biotin oder Biotien; einem Fluorophor oder Fluorophoren, einem Funküberträger oder Funküberträgern oder einer chemischen Vorstufengruppe oder Liganden gepfropft ist.

30. Gepfropftes homodetisches Cyclopeptid nach Anspruch 25, **dadurch gekennzeichnet, dass** es auf einer Seite mit B-abhängigen Epitopen vom Typ Kohlenhydrat oder T-abhängigen Epitopen und einem Immunadjuvanten gepfropft ist.

31. Therapeutische oder diagnostische Zusammensetzung, **dadurch gekennzeichnet, dass** sie ein nach Anspruch 25 gepfropftes homodetisches Cyclopeptid umfasst.

32. Verwendung eines Cyclopeptids nach Anspruch 25 oder einer Zusammensetzung nach Anspruch 31 zur Herstellung eines Arzneimittels zur Behandlung von Krebs.

33. Verwendung eines Cyclopeptids nach Anspruch 25 oder einer Zusammensetzung nach Anspruch 31 zur Herstellung eines Werkzeugs zur Diagnose von Krebs.

34. Verwendung eines Cyclopeptids nach Anspruch 25 oder einer Zusammensetzung nach Anspruch 31 zur Diagnose der Neoangionese.

35. Verwendung eines Cyclopeptids nach Anspruch 25 oder einer Zusammensetzung nach Anspruch 31 zur Suppression der Neoangionese.


### SEQUENCE LISTING

<110> Centre National de la Recherche Scientifique
   Université Joseph Fourier
   Institut National de la Santé et de la Recherche Médicale
<120> Synthèse et caractérisation de nouveaux systèmes de guidage et de vectorisation de molécules d'intérêt thérapeutique vers des cellules cibles
<130> 27117/PCT
<140> PCT/FR03/02773
   <141> 2003-09-19
<150> FR 02/11614
   <151> 2002-09-19
<150> US 60/411,845
   <151> 2002-09-19
<160> 4
<170> PatentIn version 3.1
<210> 1
   <211> 5
   <212> PRT
   <213> unidentified
<220>
   <221> PEPTIDE
   <222> (1)..(5)
   <223> Peptide circulaire dans lequel la phénylalanine en position 4 est l'isomère D
<400> 1
   Arg Gly Asp Phe Lys
   1               5
<210> 2
   <211> 5
   <212> PRT
   <213> unidentified
<220>
   <221> PEPTIDE
   <222> (1)..(5)
   <223> Peptide circulaire dans lequel la tyrosine en position 4 est l'isomère D
<400> 2
   Arg Gly Asp Tyr Lys
   1               5
<210> 3
   <211> 8
   <212> PRT
   <213> unidentified
<220>
   <221> PEPTIDE
   <222> (1)..(8)
   <223> Peptide apotogène
<400> 3
   Lys Leu Ala Lys Lys Leu Ala Lys
   1               5
<210> 4
   <211> 10
   <212> PRT
   <213> unidentified
<220>
   <221> PEPTIDE
   <222> (1)..(10)
   <223> Peptide circulaire des figures 3 et 4
<400> 4
   Pro Gly Lys Lys Lys Pro Gly Lys Lys Lys
   1               5                   10